(19) Europäisches Patentamt
European Patent Office
Office européen des brevets

(11) Publication number: **0 505 817 A1**

(12) **EUROPEAN PATENT APPLICATION**

(21) Application number: **92104039.0**

(51) Int. Cl.⁵: **A61K 31/66**

(22) Date of filing: **10.03.92**

Priority: 150391 IT PD91000055.

(30) Priority: **15.03.91**

(43) Date of publication of application:
**30.09.92 Bulletin 92/40**

(84) Designated Contracting States:
**AT BE CH DE DK ES FR GB GR IT LI LU MC NL PT SE**

(71) Applicant: **FIDIA S.p.A.**
**Via Ponte della Fabbrica 3-A**
**I-35031 Abano Terme (Padova)(IT)**

(72) Inventor: **Della Valle, Francesco**
**Via Cerato, 14**
**I-35100 Padova(IT)**
Inventor: **Romeo, Aurelio**
**Viale Ippocrate 93**
**I-00161 Rome(IT)**
Inventor: **Monastra, Giovanni**
**Via Pietro Selvatico, 84**
**I-35100 Padova(IT)**

(74) Representative: **Gervasi, Gemma et al**
**NOTARBARTOLO & GERVASI Srl Viale Bianca Maria 33**
**I-20122 Milan(IT)**

(54) **Use of phosphatidylserine and its derivatives for the manufacture of a medicament for the treatment of degenerative pathologies also associated with immune disfunctions.**

(57) The present invention relates to a new use of phosphatidylserine and of several analogous chemical compounds in various degenerative pathologies, also associated with autoimmune disfunctions wherein the tumor necrosis factor (TNF) is involved. The innovation of the use of the present invention is particularly relevant for the pharmacological therapy of pathologies such as multiple sclerosis (MS) and of other various (physiopathologic) phenomema, wherein the involvement of the above mentioned factor has been ascertained.

EP 0 505 817 A1

The present invention relates to a new use of phosphatidylserine and of several analogous chemical compounds characterized by the presence of the phosphoryl-ethanol aminic residue, and more precisely of compounds of formula:

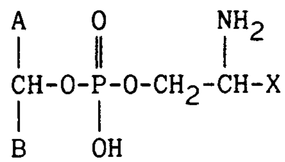

(I)

wherein X = H,-COOH; A = H,-$(CH_2)_n$-O-CO-R with n = 1,2,

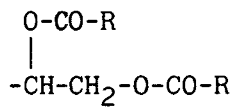

and B = -$(CH_2)_m$-OY with Y = H or -CO-R and m = 1,2,3,4,

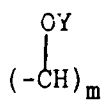

-$CH_2$ -OY with Y = H or -CO-R and m = 1,2,3 provided that when A is different from H, m = 1,2 and wherein the different groups R may be equal or different from each other and the corresponding -CO-R groups represent acyl radicals of acids of the aliphatic, aromatic, alicyclic or heterocyclic series, or of a mixture of compounds corresponding to the above mentioned formula but differing from each other for the different meaning of R, when radical -CO-R derives from higher aliphatic acids which are normally present in natural phospholipids, or of their acid addition salts or of their metal salts.

According to the present invention said compounds or mixtures of compounds may be used in the therapy of different degenerative pathologies also associated with immune disfunctions wherein the so called "tumor necrosis factor" (TNF) is involved.

The innovation of the therapeutic use of the present invention is particularly relevant for the pharmacological therapy of pathologies such as multiple sclerosis (MS) and of other various (physiopathologic) phenomena wherein the involvement of the above mentioned factor has been ascertained.

## BACKGROUND OF THE INVENTION

A. Tumor necrosis factor (TNF).

Tumor necrosis factor is a cytokine having a proteic structure, formed by 156-157 aminoacids, depending on the specific animal species. TNF, in the cell, derives from a precursor of 233-235 aminoacids: for example in man TNF is produced as a propeptide (inactive form of about 233 aminoacids) from which a mature residual protein is obtained (active soluble form of 157 aminoacids, that is molecular weight of 17 kiloDaltons). Therefore TNF is in the form of dimer, trimer, tetramer or pentamer; the form corresponding to 17 kiloDaltons (kD) was never isolated (Beutler B.: Tumor necrosis, cachexia, shock and inflammation: a common mediator. Ann. Rev. Biochem. 57: 505-518, 1988; Tracey K. J. et al.: Cachectin/tumor necrosis factor. Lancet May 20, 1122-1125, 1989). Two different types of TNF were identified namely $\alpha$-TNF and $\beta$-TNF. TNF is produced by different cells, in particular, TNF-$\alpha$ is secreted by macrophages, T lymphocytes, mastocytes, astroglia, microglia, Kupffer cells, non-striated muscular cells; on the contrary TNF-$\beta$ is secreted only by T-lymphocytes (Beutler B.: Tumor necrosis, cachexia, shock and inflammation: a common mediator. Ann. Rev. Biochem. 57: 505-518, 1988; Steffen M. et al.: Simultaneous production of tumoral necrosis factor and lymphotoxic by normal T cells after induction with IL-2 and anti-T3. J. Immunol. 140, 2621-2624, 1988; Gordon J. R. et al.: Mast cells as a source of both performed and immunologically inducible TNF-$\alpha$ cachectin. Nature 346, 274-276, 1990).

Furthermore, all the cells, with the exclusion of the erythrocytes, possess a receptor for TNF. Said

factor participates to the immunomodulation humoral mechanisms enhancing the defensive reactions.

However, TNF does not only show an antitumoral activity, as it was supposed until about ten years ago, but it is also a multifunctional molecule (similarly to other cytokines) with pleiotropic effects, in particular:

- inhibition (for example: at lipoproteinlipase level, tumoral cells growth) (Carswell, E.A. et al.: An endotoxin-induced serum factor that causes necrosis of tumors. Proc. Natl. Acad. Sci. 72; 3666-3670, 1975)
- stimulus (for example: mitogen for fibroblasts, epithelial cells, astrocytes) (Selmaj, K.W. et al.: Proliferation of astrocytes in vitro in response to cytokines. J. Immunol. 144, 129-135, 1990).
- modulation of various processes which the organism well on in years is restored by, (for example: remotion of the advanced glycosylation products as far as collagen "remodeling" is concerned) (Vlassara H. et al.: Cachectin/TNF and IL-1 induced by glucose-modified proteins: role in normal tissue remodeling. Science 240, 1546-1548, 1988).

It is described in literature that invasive stimuli such as infections, neoplastic proliferation and rejection diseases provoked by transplants ("graft versus host disease") induce the production of TNF-$\alpha$ and $\beta$ - (Steffen et al.: Simultaneous production of tumor necrosis factor-and lymphotoxic by normal T cells after induction with IL-2 and anti-T3. J. Immunol. 140, 2621-2624, 1988). The effect of this cytokine is to increase the immune and inflammatory reaction, by promoting the activation of neutrophils, the secretion of interleukin-1 (IL-1) and by inducing a "helper" signal for the activation of T-cells in combination with interleukin-6 (IL-6) (Kihweide, R. et al.: Tumor necrosis factor-and interleukin 6 systemically induce T cell growth. Eur. J. Immunol. 20, 1019-1025, 1990). Under normal conditions, TNF is absent in the organism, the cells producing it contain only mRNA for TNF, but not the protein, which is synthesized only at the moment of the stimulus.

However, under non physiological conditions, namely under excessive TNF-$\alpha$ secretion conditions seriously toxic and various morbid phenomena are observed. In literature it is also described that intravenous administrations of TNF reproduce the symptoms of the endotoxic shock, whereas chronic administrations reproduce the cachectic state, characteristic of the terminal phases of neoblastic diseases and of serious infections (Beutler B.: Tumor necrosis, cachexia, shock and inflammation: a common mediator. Ann. Rev. Biochem. 57: 505-518, 1988). For this reason TNF is also known with the name cachectin.

It is important to note that the excessive TNF secretion is involved in a series of morbid phenomena: pneumonic fibrosis (Piquet, P.F. et al.: Tumor necrosis factor/cachetin plays a key role in bleomycin-induced pneumopathy and fibrosis. J. Exp. Med. 170, 655-663, 1989), malaria cerebral complications (Grau G.E. et al.: Tumor necrosis factor (cachectin) as an essential mediator in murine cerebral malaria. Science 237: 1210-1212, 1987), insuline-dependent diabetes (Poulsen, T.M. et al.: Human tumor necrosis factor potentiates human interleukin-1 mediated rat pancreatic $\beta$-cell cytotoxicity. J, Immunol. 139, 4077-4082, 1987), bacterial infections (Waage A. et al.: Association between tumor necrosis factor in serum and fatal outcome in patients with meningococcal disease. (Lancet February 14, 355-357, 1987), protozoal infections (Scuderi P. et al.: Raised serum levels of tumor necrosis factor in parasitic infections. Lancet, December 13, 1364-1365, 1986), HIV infections, namely "human immonodeficiency virus" (Hober D. et al.: Production of tumor necrosis factor-alpha (TNF-alpha) and interleukin-1 (IL-1) in patients with AIDS. Enhanced level of TNF-alpha is related to a higher cytotoxic activity. Clin. Exp. Immunol. 78 (3) 329-333, 1989), arthritis (Di Giovine F. S. et al.: Tumor necrosis factor in synovial exudates. Ann. Rheumatic Diseases 47, 768-772, 1988), cachectic states associated with infections and neoplastic diseases (Beutler B.: Tumor necrosis, cachexia, shock and inflammation: a common mediator. Ann. Rev. Biochem. 57: 505-518, 1988) and multiple sclerosis (Selmaj K. W. et al.: Tumor necrosis factor mediates myelin and oligodendrocyte damage in vitro. Ann. Neurol. 23: 339-346, 1988).

B. Multiple sclerosis (MS) and TNF

A particular attention must be paid to multiple sclerosis and to the possibility of an innovative therapeutical intervention for treating this disease. Multiple sclerosis (MS or sclerose en plaque) is one of the most important "demyelinizing" disease, meaning with such an anatomo-pathologic terminology, a morbid condition wherein the process affects mainly the white matter (myelin sheath destruction), whereas the axial fiber is rather in a good state of preservation.

MS, described for the first time in 1868 by Charcot, is probably the most frequent neurologic disease occurring to the young adult. It usually begins between 18 and 35 years (but it is not rare a later beginning between 35 and 55 years). The course is progressive and characteristic, in fact, even though in the general pattern of a graduated worsening, periods of spontaneous improvement occur (some times up to complete

regression of the symptomatology), alternated with periods of serious worsening (poussées course).

After some years a serious motor disability is usually observed, the exitus may occur after very many years from the disease beginning.

Sometime the course is much more benign (MS slight form), with complete well being and modest late disability.

The anatomo-pathologic pattern of this disease is characterized by the presence of lesions, namely of "plaques", at the expense of the central nervous system with primary involvement of the white matter (namely of myelinic fibers). These plaques are in fact characterized by demyelination areas, with successive glial reaction, having mostly an irregular shape and a volume ranging from some millimeter fraction to some centimeters.

In CNS the plaques are spread plentyfully and ubiquitously in the white matter, while the gray matter is substantially unimpaired.

Some sites seem to be involved more frequently: among them the bulb, the corpus callosum, the white matter around ventriculi cerebri, the bridge, the central region of the mesencephalon the cervical and dorsal segments of medulla, etc.

In addition, inside the nervous substance, the plaques are more frequently found in the perivasal site.

The most typical feature of the above pattern and, which occurs first, resides in the degeneration of myelinic sheaths of the nervous fibers comprised in the plaques.

Firstly myelin swells up, after splits up, taking on a well defined coloration with osmic acid. The fragments are then phagocytized little by little by cells having phagocytal functions, most probably the microglia cells.

Together with the myelinic involvement, a pain occurs, although minor and unsteady at the axial fibers. These ones in fact are nearly never interrupted but only deformed, thereby appearing uniformly thickened, or with occasional swellings. The glial reaction is secondary with respect to the demyelination process.

The involvement of the blood vessels around the plaques, at their periphery, and in their inside is nearly steady and massive.

Passive congestion and perivascular edema, walls thickening caused by parietal infiltrates, and thrombotic phenomena are often observed; infiltrations of histocytic and lymphocytic series elements, and plasma cells are present in the perivascular sites at the plaques periphery.

The variety of the symptomatologic pattern induced some authors to distinguish various clinical forms.

The difference on the basis of the onset conditions and the course rapidity is the most important and significant: we can therefore distinguish between an acute and a chronic form.

The first one begins abruptly with a symptomatology at once massive, consisting of lower extremities hemiplegia, monoplegia or paraplegia or paralysis of more cranical nerves or a jacksonian epilepsy crisis, or more often with some of the above symptoms contemporaneously.

The course, although being typically a poussées is quickly ingravescent. The pattern is therefore very similar to that of acute perivenous postexanthematous and post-vaccinal encephalomyelitis.

The chronic intermittent form of multiple sclerosis is the most frequent (60 % of the cases), with insidious beginning, a course characterized by exacerbations and periods of complete or partial symptomatology regression; after twenty years from the disease beginning, many patients, probably the half of the cases, are unable to walk; the exitus may occur after many years provoked by pneumonic and urinary tract infections, bedsore or more rarely by cardiorespiratory centers involvement.

The etiopathogenesis of this disease is still unknown: in fact the underlying mechanism to the myelinic damage in MS is mostly speculative, anyway the immune factors seem to play a primary role (Waksman B.: Mechanisms in multiple sclerosis. Nature 318: 104-105, 1985).

MS plaques in course of demyelination are infiltrated by macrophage and lymphocytes (Traugott U.: Characterization and distribution of lymphocyte subpopulations in multiple sclerosis plaques versus autoimmune demyelinating lesions. In Semin. Immunopathol. 8: 71-95, 1984), but the pathogenic meaning of these infiltrates remains unknown.

It was demonstrated in vitro that products of immune system may damage myelin.

For example antigalactocerebroside antibodies participate to the demyelination of cultures coming from central nervous system tissues (Raine C. S. et al.: Demyelination in vitro: absorption studies demonstrate that galactocerebroside is a major target. J. Neurol. Sci. 52: 117-131, 1981), but up to now convincing evidences do not exist about the role of said antibodies in MS.

Mononuclear cells may also induce demyelination in vitro (Lyman A. D. et al.: Antigen-specific T cells can mediate demyelination in organotypic central nervous system cultures. Cell Immunol. 102: 217-226, 1986), but it was not yet ascertained exactly which cytotoxic mechanisms are involved.

In the last years, a particular attention was paid to cytolytic mechanisms of the histic damage caused

by MS, and especially to the possible role of cytokines more precisely of the tumor necrosis factor (TNF).

In fact in vitro experimental data show that:

- TNF-α is produced by astrocytes stimulated by a lypopolysaccharide (LPS) (Chung I. Y. et al.: Tumor necrosis factor-a production by astrocytes. Induction by lipopolysaccharide, IFN-gamma and IL-1β[1]. J. Immunol. 144: 8, 2999-3007, 1990).
- TNF-α is also produced by microglial cells when previously exposed to an endotoxin (LPS) and interferon-γ (IFN γ) (Frei K. et al.: Antigen presentation and tumor cytotoxicity by interferon-gamma treated microglial cells. Eur. J. Immunol. 17: 1271-1278, 1987).
- TNF is mytogen for the astrocytes (Selmaj K. W. et al.: Proliferation of astrocytes in vitro in response to cytokines. A primary role for tumor necrosis factor. J. Immunol. 144: 1, 129-135, 1990), it causes myelinic sheath dilation and it is cytotoxic for the oligodendrocytes, (Robbins D. S. et al.: Production of cytotoxic factor for oligodendrocytes by stimulated astrocytes. J. Immunol. 139: 2593-2597, 1987; Selmaj K. W. et al.: Tumor necrosis factor mediates myelin and oligodendrocyte damage in vitro. Ann. Neurol. 23: 339-346, 1988).

Moreover, as a confirmation of the hypothesis about TNF involvement in MS, in vivo experimental data show that treatments with anti-TNF antibodies prevent the onset of "adoptive transfer" autoimmune allergic encephalomyelitis (AAE) in mouse (Ruddle N. H. et al.: An antibody to lymphotoxin and tumor necrosis factor prevents transfer of experimental allergic encephalomyelitis. J. Exp. Med. 172: 1193-1200, 1990). This AAE pattern is particularly interesting since it is considered a valid animal pattern of MS (Pettinelli C. B. et al.: Adoptive transfer of experimental allergic encephalomyelitis in SJL/J mice after in vitro activation of lymph node cells by myelin basic protein: requirement for $Lytl^{+}2^{-}$ T lymphocytes. J. immunol. 127: 1420-1423, 1981).

A further confirmation of the hypothesis about the TNF role in MS can be deduced by clinical studies data indicating:

- the presence of TNF in astrocytes and cerebral macrophages belonging to patients deceased by MS (Hofman F. M. et al.: Tumor necrosis factor identified in multiple sclerosis brain. J. Exp. Med. 170: 607-612, 1989).
- high and abnormal levels of TNF produced by macrophages (stimulated by LPS) taken from blood samples of patients affected by MS (Beck J. et a].: Increased production of gamma interferon and tumor necrosis factor precedes clinical manifestation in multiple sclerosis: Do cytokines trigger off exacerbation? Acta Neurol. Scand. 78: 318-323, 1988).
- high cytokines levels (such as interleukin-1β and TNF) in MS patient's cerebrospinal fluid (Hauser S. L. et al.: Cytokine accumulations in CSF of multiple sclerosis patients: Frequent detection of interleukin-1 and tumor necrosis factor but not interleukin-6. Neurology 40: 1735-1739, 1990).

As far as the hypothesis about the mechanism of TNF action in MS is concerned, one could suppose that TNF plays a central role during the first phases of said disease, and probably also in the period preceding the relapses, exercising an effect on myelinic and axon membranes which brings to a hydration imbalance.

The alterations caused by TNF in myelinic sheath could be explained in view of the documented effects of TNF on water and electrolytes transfer through the membranes (Beutler B. et al.: Cachectin: more than a tumor necrosis factor. New Engl. J. med. 316: 379-385, 1987, Le And J. et al.: Biology of disease. Tumor necrosis factor and interleukin 1: cytokines with multiple overlapping biological activities. Lab. Invest. 56: 234-248, 1987) and the TNF capacity to reduce the muscular cells transmembrane potential (Tracey K. J. et al.: Cachectin/tumor necrosis factor mediates change in skeletal muscle plasma membrane potential. J. Exp. Med. 163: 1368-1373, 1986).

The preceding experimental and clinical data relating to the individual connections between TNF and MS are thus to be considered as a confirmation of this hypothesis.

In view of the foregoing and because of the significance of this pathology the importance is comprehensible of identifying MS pharmacological therapies.

In fact, at present, a real therapy of MS, namely being able to prevent exacerbation and to stop the course progressivity, does not exist.

The therapy, encompassing the pre-treatment with corticosteroids, is to be used for short periods (2-3 weeks) at the first sign of the disease recrudescence: in fact in controlled clinical studies a beneficial effect was found on the disease acute phase in many (not all) patients, by shortening the exacerbation period, but not modifying the disease progression.

Also the effectiveness of other therapies such as those encompassing the use of immunosuppressive drugs is still discussed.

SUMMARY OF THE INVENTION

The application of derivatives of the present invention in the above discussed pathological conditions, associated with an excessive TNF secretion, has the effect to reduce TNF plasmatic levels, as it will be shown later on for the case of phosphatidylserine (BC-PS described in EP 0148045 granted on 29.06.88) with mice and rabbits experiments, to prevent the onset of autoimmune encephalitis or to reduce significantly its disabling evolution.

It is known that the PS administered in vivo can follow at least two different pathways (decarboxylase with formation of PE and deacylation with formation of lysocompounds) - Palatini et al. Bri. J. Pharm. 1991, 102, 345-350.

These results, indicating an inhibitory effect of derivatives of formula (I) on cells secreting TNF are of considerable utility from a clinical point of view for an innovative therapeutical use in degenerative pathologies also associated with auto immune disfunctions wherein the tumor necrosis factor is involved.

Among said diseases multiple sclerosis cachectic states associated with infections and neoplastic diseases, pneumonic fibrosis, arthritis deformants, bacterial, protozoal and HIV (human immunodeficiency virus) infections, malaria cerebral complications, insulino-dependent diabetes are to be cited.

The study reported hereinbelow highlights:

1. Effect of BC-PS on seric levels of TNF, induced by bacterial lipopo]ysaccharides (LPS) in small mice, and rabbits.
2. Effects of BC-PS in animal pattern of multiple sclerosis: neurologic-behaviouristic, histopathologic and biochemical evaluations (TNF levels) in an experimental allergic encephalitis (EAE) in mice.

Relating to point 1:

Effect of BC-PS on TNF seric level induced by bacterial lipopolysaccharide (LPS) in small mice and rabbits.

By the experimentation described hereinbelow the effectiveness of the treatment with BC-PS (i.p. and i.v., vs other phospholipids) is evaluated by reducing the formation of tumor necrosis factor (TNF), induced by a specific exogenous stimulus such as the lipopolysaccharide of bacterial origin (LPS) injected by parenteral route both in mice and in rabbits.

Experimental procedures

Animals and experimental pattern

Utilized are:

- 170 Charles River CD-1 albino male mice weighing about 25-30 g
- 6 New Zealand male rabbits weighing about 2-2.5 kg.

The animals after 90' from the last phospholipidic treatment or with THIS (as indicated later-on), are treated by parenteral route with S. Thyphi or E. Coli (Sigma) lipopolysaccharide.

Particularly:

- mouse: injection of 5 µg LPS i.p. ($1^{st}$ experiment) or 25 µg LPS i.v. ($2^{nd}$ experiment) the blood sample is taken after 60 min.
- rabbit: injection of 1 µg LPS i.v.. The blood sample is taken after 90 minutes.

Tested products and pharmacological treatment

The following products are tested:

- BC-PS (phosphatidylserine in the commercially available form consisting of a composition comprising: phosphatidylserine extracted from bovine brain as described in example 1)
- PS (phosphatidylserine)
- PC (phosphatidylcholine)
- PG (phosphatidylglycerol)
- DPS (lysophosphatidylserine)
- PE (phosphatidylethanolamine)
- $PPS_4$ (1-myristoyl-2-deoxi-3-glycerophospho-L-serine)

The phospholipids purity and their composition are determined by thin layer bidimensional chromatography (Punzi, L. et al.: Phospholipids in inflammatory synovial effusions. Rheumatol. Int. 6, 7-11, 1986).

Phospholipids are then solubilized in 50 mM TRIS-HCl pH 7.8 and suitably sonicated.

The phospholipidic dispersion is then injected in animals under the following conditions:

- mouse: BC-PS 15-30 mg/kg/die i.p. for 3 days or 30 mg/kg/die i.v. for 3 days DPS, $PSS_4$ 20

mg/kg/die i.p. for 3 days PS, PC, PG, PE 25 mg/kg/die i.p. for 3 days

- rabbit: 30 mg/kg/die i.v. for 5 days.

LPS is then administered following the above mentioned conditions with successive blood sample for the seric TNF dosage.

The level of seric TNF was evaluated by biological dosage, using the fibroblasts line L-929 of murine sarcoma (American type culture collection). In fact TNF is cytotoxic against said line (Meager A. et al.: Assays for tumor necrosis factor and related cytokines. J. Immunol. Methods 116: 1-17, 1989).

Shortly, 5 x $10^4$ cells are cultured in 96 well plates with 0.1 ml RPMI 1640 medium containing 10 % (v/v) of bovine serum inactivated at 56 °C for 30'.

After 18 hours the medium is replaced with 0.1 ml of fresh medium, containing serial dilutions of serum samples to be tested in the presence of actinomycin D (2 μg/ml) and lithium chloride 20 mM in order to increase the method sensitivity, namely the TNF cytotoxicity (Beyaert R. et al.: Lithium chloride potentiates tumor necrosis factor-mediated cytotoxicity in vitro and in vivo. Proc. Natl. Acad. Sci. 86: 9494-9498, 1989).

An anti-TNF antibody (4000 U/ml of TNF-α(Genzyme) is added to 3 wells in every plate and said wells are utilized as positive control in order to check the sensitivity level of the cellular line, namely to verify the specificity dosage.

As negative controls, wells containing untreated mice serum, are used.

After 18 hours the medium is sucked and replaced with 0.1 ml of a saline solution containing 30 mM of glucose and 0.45 mg/ml of MTT tetrazolium (3-[4,5-dimethylthiazol-2-yl]-2,5 diphenyl-tetrazolium bromide. Then, after 4 hours, 0.1 ml of an acid solution of isopropanol (0.08 N HCl in isopropanol) is added and the absorbance is read by using a plate spectrophotometer scanner provided with a filter specific for 570 nm absorbance.

TNF cytotoxicity is calculated, by applying the formula:

$$\%\ \text{cytotoxicity} = 1 - \frac{\text{absorbance(treated)} - \text{absorbance(rTNF-}\alpha)}{\text{absorbance(control)} - \text{absorbance(rTNF-}\alpha)}$$

TNF-α title in serum samples is expressed in unit/ml and corresponds to the inverse of the dilution bringing to 50 % of cellular survival (Meager A. et al.: Assays for tumor necrosis factor and related cytokines. J. Immunol. Methods 116: 1-17, 1989).

In order to validate the test an anti-TNF-α murine antibody (Genzyme) is used which, as foreseen (see the results) blocks completely the cytotoxicity of murine serum but not the one of rabbit serum.

## RESULTS

In fig. 1 reported are the results relating to the effect of i.p. and i.v. pretreatment with BC-PS on TNF secretion of respectively mice (A) and rabbits (B), evaluated by means of the relating cytotoxicity on cellular cultures.

It is evident that the pretreatment with BC-PS reduces TNF production, induced by LPS (respectively 5 μg i.p. and 1 μg i.v.) in both cases, in particular it is observed:

- in mice about 81 % inhibition
- in rabbits about 89 % inhibition.

Figure 1A: BC-PS is administered i.p. at the dose of 30 mg/kg/die for 3 days, to groups of 6 mice. TNF-α secretion is then induced by i.p. injection (5 μg) of S. Thyphi LPS,after 90' from the last BC-PS injection. The blood was taken 60' later.

Figure 1B: BC-PS was administered at the dose of 30 mg/kg/die by i.v. route for 5 days to groups of 3 rabbits . TNF secretion is then induced by i.v.injection (1 μg) of E.Coli LPS, after 90' from the last BC-PS injection, blood samples were taken 90 ' later. Said data are the average values ± SEM of the seric cytoxicity measured on cells L-929 (see experimental procedures).

In table 1 reported are further results ($2^{nd}$ experiment) relating to the i.v. and i.p. effect of the pre-treatment with BC-PS in the mouse and successive i.v. administration of 25 μg LPS. The data confirm BC-PS effectiveness, in particular the inhibition of cytotoxicity and therefore of TNF production) is about 64 % and 72 % after respectively i.v. and i.p. administration.

Further data (not reported) demonstrate that BC-PS effectiveness already appears after 2 administration days, and is completely evident at the third day, whereas no further improvement is observed if said pretreatment is extended up to 10 days.

BC-PS effectiveness remains for at least 2 days from the last injection, while the inhibitory effect on TNF release disappears completely after five days.

In table 2 reported are the data indicating the BC-PS dose/effectiveness and the comparison with other phospholipids effect. It is evident that:

- BC-PS has a maximum inhibition 77% at the dose of 30 mg/kg i.p., whereas the effect is lower at 15 mg/kg (37% inhibition);
- phosphatidylserine (PS), lysophosphatidylserine (DPS) and phosphatidylethanolamine (PE) are active (respectively 68-80-56% inhibition at the dose of 20-25 mg/kg); a very slight inhibition is observed in the presence of phosphatidylcholine (PC) (16%), whereas phosphatidylglycerol (PC) is inactive;
- the derivative 1-mirystoyl-2-deoxi-3-glycero-L-serine ($PSS_4$) is active by reducing (about 82%) the seric levels of TNF-α.

The results reported in Table 2 show that the administration of DPS (lysophosphatidylserine) or PE (phosphatidylethanolamine) are also able to lower the circulating TNF levels.

This shows that the PS metabolites (decarboxylate products and corresponding deacylate products) are also able to produce a pharmacological effect in the pathologies, in which the TNF is involved.

Table 1

| Inhibition of TNF-α production induced by LPS in the mouse : effect of intraperitoneal and intravenous treatment with BC-PS | | | |
|---|---|---|---|
| Treatment | phospholipid admin. route[a] | TNF-α title (Unity/serum ml) | Inhibition (%) |
| LPS[b] buffer | i.v. (6) | 543 ± 79[c] | |
| BC-PS LPS | i.v. (5) | 195 ± 97 | 64 |
| LPS buffer | i.p. (5) | 230 ± 36 | |
| BC-PS LPS | i.p. (6) | 65 ± 16 | 72 |

a. BC-PS is administered i.p. at the dose of 30 mg/kg/die/ or i.v. for three days
b. LPS (25 µg i.v.) was administered after 90' from the last BC-PS injection, then 60' later the blood sample was taken in order to determine the TNF-α title.
c. The data correspond to the average ± SEM (the number of mice is indicated between brackets).

Table 2

| inhibition of TNF-α production in the mouse, induced by LPS: dose-effect phospholipids PS, PC, PG, PE, DPS, $PSS_4$ i.p. administered. | | | | |
|---|---|---|---|---|
| phospholipide | dose[a] (mg/kg) | TNF-α title (U/serum ml)[b] | | Inhibition (%) |
| | | Controls | treated | |
| BC-PS | 15 | 254±25[b] (7) | 161±26 (6) | 37 |
| BC-PS | 30 | 213±23 (7) | 49±14 (6) | 77 |
| PS | 25 | 252±59 (9) | 81±21 (9) | 68 |
| PC | 25 | 210±38 (12) | 176±53 (12) | 16 |
| PG | 25 | 180±25 (18) | 238±37 (18) | -- |
| PE | 25 | 313±42 (6) | 137±46 (6) | 56 |
| DPS | 20 | 225±30 (5) | 45±11 (5) | 80 |
| $PSS_4$ | 20 | 277±38 (5) | 50±16 (5) | 82 |

a. BC-PS and the other phospholipids are i.p. administered for 3 days. LPS from S. Thyphi (5 µ i.p.) is injected after 90' from the last phospholipid injection: the blood sample is taken 60' later.
b. The data correspond to the average ± SEM (the number of mice is indicated between brackets).

Referring to item 2:

Effect of BC-PS in an animal pattern of multiple sclerosis: clinical, hystopathologic and biochemical evaluations (TNF levels) in an experimental allergic encephalitis AAE in mice.

By the experimentation described hereinbelow the effectiveness of the pharmacological treatment with BC-PS is evaluated on the autoimmune allergic encephalitis (AAE) pattern.

Said experimental pattern, realized on mice according to Pettinelli et al. (Pettinelli C. B. et al.: Adoptive transfer of experimental allergic encephalomyelitis in SJL/J mice after in vitro activation of lymph node cells by myelin basic protein: requirement for Lyt $1^+$ $2^-$ T lymphocytes. J. Immunol. 127, 4, 1420-1423, October 1981), represent as described when discussing the background of the invention, a valid animal pattern of multiple sclerosis, characterized by inflammatory phenomena of the central nervous system.

In particular with the experimentation described hereinbelow it was determined that BC-PS is effective respectively in

- blocking the disease onset
- reducing the disease seriousness and progression.

## Experimental procedures

### Animals and AAE induction

26 healthy female 6-10 weeks old mice of the strain SJL/J (H-$2^S$),obtained from Jackson Laboratories (Bar Harbor, ME) are utilized.

AAE is induced by injection of lymph node cells (LNC 8 x $10^7$) sensitized with myeline basic protein (MBP) according to the methodology described by Pettinelli (Pettinelli et al.: J. Immunol. 127, 1420-1423, 1981). The lymph node cells come from healthy donors immunized with MBP.

Briefly, about 20 healthy donor female mice (SJL/J strain) are immunized by subcutaneous route with 0.4 mg/mouse of MBP in complete Freund adjuvant (CFA) (MBP is prepared from guinea pig myelon (Rockland, Gilbertville, PA) according to the procedure described by Deibler et al (Deibler, G.E. et al.: large scale preparation of myelin basic protein from central nervous system tissue of several mammalian species. Prep. Biochem. 2:139-151, 1972).

Then after 10 days from immunization, the mice thus immunized are suitably sacrificed in order to take limph node samples. Limph node cells (LNC), obtained by mechanic disgregation on metallic sieves, are then counted, evaluating their cellular vitality by means of Trypan blue, and scattered in 24 wells plate (2 ml/plate) at the density of 8 x $10^6$ cells/well in the presence of 50 $\mu$g MPB/ml in RPMI 1640 (Sigma Chemical Co, St. Louis, MO), with addition of 10 % FCS, inactivated at 56 °C for 30', penicillin (100 U/ml, Sigma), Streptomycin (100 $\mu$g/ml, Sigma), non essential aminoacids, sodium pyruvate, $\beta$-mercaptoethanol and glutamine.

After an incubation period of 3 days at 37 °C in an incubator (5 % $CO_2$), the cells are collected (with sterile Pasteur), washed twice with the specific medium above described (in order to ensure MBP removal) and suspended in Hanks balanced saline solution (Sigma).

The cells, after being suitably counted and their cellular vitality being evaluated, are injected in mice at the concentration of 8 x $10^7$ cells/100 $\mu$l/mouse by intravenous route (caudal vein). The disease normally sets in after 5-10 days.

### Substance under examination and relating pharmacological treatment.

BC-PS is dissolved in Tris buffer, 50 mM pH 7.8 then sonicated and injected by intraperitoneal route (i.p.) at the dose of 30 mg/kg/die in a volume of 150-200 $\mu$l (0.1 ml/10 g of body weight)

The treatment is prolonged for the entire experiment period (first experiment 60 days, second experiment 40 days); the first administration is carried out 6 hours after injection of LNC, sensitized with MBP.

The control animals are daily treated with Tris buffer 50 mM, pH 7.8 by i.p. route with the same volume/weight ratio used for BC-PS treatments.

The following Table 3 reports schematically the total number (n) of treated and control animals for the two individual experiments conducted.

Table 3

| Experiment | Control (Tris) | Treated (BC-PS) |
|---|---|---|
| 1st (n = 15)<br>2nd (n = 11) | n = 6**<br>n = 5 | n = 9*<br>n = 6 |

wherein:
* on 4 animals the BC-PS treatment is suspended at the 14th day from EAE induction (by LNC/MP injection)
** the chronic treatment with BC-PS is started out at the 16th day on 3 control animals under clear EAE condition (and it is always carried on until the 60th day from EAE induction).

On the ground of what above reported the BC-PS therapeutic regimen can be summarized as follows:

```
                 EAE ind.
                 °         BC-PS 30 mg/kg/die i.p.
1st regimen    ______________________________________________
(n=11)             6 hr.                                60 days

                 EAE ind.
                 °         BC-PS 30 mg/kg/die i.p.  TRIS
2nd regimen    ______________________________________________
(n=4)              6 hr.          14 days               60 days

                 EAE ind.
                 °   TRIS                BC-PS 30 mg/kg/die i.p.
3rd regimen    ______________________________________________
(n=3)                             16 days               60 days
```

Parameters

The following evaluations were carried out
1. neurologic-behaviouristic
2. histopathologic
3. biochemical
In particular:

1. Neurologic behaviouristic evaluations

The neurologic defects are registered in blind according to the following evaluation scale
0 = no abnormality
1 = flabby tail, with initial debility of hind arms
2 = flabby tail with moderate debility of hind arms
3 = one hind arm paresis
4 = total hind arms paresis
5 = quadriplegia - cachectic state, death
The observation period is prolonged until 60 days from EAE induction

2. Histopathologic evaluations

The morphologic evaluation is carried out on a total of 5 animals and among them:

- 2 are the control (TRIS)
- 2 are treated with BC-PS and then with TRIS (on the base of the $1^{st}$ regimen.
- 1 is treated with BC-PS and then with TRIS (on the base of the $2^{nd}$ regimen).

The animals, are sacrificed, under anesthesia conditions, after different time Periods (after 7-11-20 days from EAE induction) and perfused with 2.5 % glutaraldehyde (by aorta route).

The nervous tissue is taken, and thin sections (0.5-1 mm) are carried out at all the neuraxis levels.

The thin slices are fixed for 1 hour in 1% cold Osmium tetroxide (preferred fixative in order to highlight myelin) then dehydrated with solutions having growing alcoholic concentrations, washed with propylene oxide and soaked in Epon 812.

The slices are then dried at increasing temperatures from 37 to 60 °C for 2 days, then cut by microtome into thin sections of 1 μ, coloured with toluidine blue and examined, in blind, on the optic microscope (0.M.).

The Inflammatory Scale (IS), comprised between 0 and 4 is determined on the basis of the inflammatory cells present in the CNS meningeal, perivascular and parenchyma compartment.

The demyelination scale (DS), comprised between 0 and 4, is determined on the basis of the number of demyelinated axons.

IS and DS, indicating respectively the inflammation and demyelination seriousness, are determined according to criteria already described in literature (Moore G. R. W. et al.: Experimental autoimmune encephalomyelitis. Augmentation of demyelination by different myelin lipids. Laboratory Investigation vol. 51, 4, 416, 1984).

Particularly:

a) the inflammatory scale (IS) is evaluated according to the following evaluation scale

0 = few inflammatory cells in leptomeninges

1 = leptomeningeal infiltrate spread around the blood vessels.

2 = large perivascular engorgements and expansion of the infiltrate in the adjacent subarachnoid space.

3-4 = large perivascular engorgement and increased subarachnoid inflammation.

b) The demyelination scale (DS) is evaluated on the basis of the following evaluation scale:

0 = few spread demyelinated subpial axons

1 = groups of subpial axons, free from myelin sheath

2 = small demyelination plaques extending beyond the subpial regions

3 = large demyelination plaques extending deeply in the white matter.

4 = large white matter involvement

3. Biochemical evaluation

TNF levels produced by lymph nodes cells and splenocytes (SC) are evaluated.

The cells are obtained respectively from the lymph nodes and from the spleen taken from 1 control animal and 1 treated with BC-PS, before glutaraldehyde perfusion (for the histologic evaluation above described). The samples and the cultures are repeated more than once, on the occasion of the various perfusions.

The cells, obtained by mechanical disaggregation with metallic sieves, are introduced into cultures in RPMI 1640 (Sigma chemical Co., St. Louis, MO), with 10 % FCS, inactivated at 56 °C for 30', penicillin (100 U/ml, Sigma), streptomycin (100 μg/ml, Sigma) non essential aminoacids, sodium pyruvate, β mercaptoethanol and glutamine.

The cells are then scattered at high density on 24 wells plate (2 ml medium/plate) ($8 \times 10^6$ cells/well = $4 \times 10^6$ cells/ml or $80 \times 10^6$ cells/well = $40 \times 10^6$ cells/ml).

After 1 incubation day at 37 °C in an incubator (5 % $CO_2$), the medium in which the cells were cultured is sucked and TNF is dosed by biologic method using the fibroblasts line L-929 of murine Sarcoma (American Type culture collection).

In fact TNF is cytotoxic against this line (Meager A. et al.: Assays for tumour necrosis factor and related cytokines. J. Immunol. Methods 116: 1-17, 1989).

Briefly, cells L-929, grown in RPMI 1640 (Sigma, St. Louis, MO) provided with sodium bicarbonate, (2g/l), gentamicin (50 μg/l and 10 % fetal calf serum inactivated for 30' at 56 °C were scattered onto 96 well plates at the density of $5 \times 10^4$ cells.

After 18 hours, the medium is replaced with 0.1 ml fresh medium, containing serial dilutions of the samples to be tested (namely of the above mentioned incubation medium of LNC and SC cells) in a total volume of 100 μl/well.

Moreover, in the medium actinomycin D (2 µg/ml/Sigma) and lithium chloride 20 mM (Sigma) are also present in order to increase the method sensitivity, namely TNF cytotoxicity (Beyaert R. et al.: Lithium chloride potentiates tumor necrosis factor-mediated cytotoxicity in vitro and in vivo. Proc. Natl. Acad. Sci. 86: 9494-9498, 1989).

An anti-TNF antibody (4000 U/ml of rTNF-$\alpha$ (Genzyme)) is added to 3 wells in each plate, utilized as positive control in order to check the sensitivity limit of the cellular line, namely to verify the dosage specificity.

As negative controls, wells are used containing LNC or SC supernatants taken from untreated mice.

The cells vitality is evaluated after 24 hours of incubation at 37 °C in $CO_2$ (5 %), replacing the RPMI 1640 medium with 100 µl of PBS containing 0.6 % glucose and 15 µl of tetrazolium salt MTT (45 µg/well; Sigma).

The colorimetric test is stopped after 4 hours by adding 8-10 times with a pipette 100 µl of acidified isopropyl alcohol (0.08 N HCl in isopropanol) until complete cell lysis and complete MIT dissolution.

The plates are then read by means of a plate spectrophotometric scanner provided with a filter specific for 570 nm absorbance.

TNF cytotoxicity was calculated by applying the formula:

$$\%\ \text{cytotoxicity} = 1 - \frac{\text{absorbance(treated)} - \text{absorbance(rTNF-}\alpha)}{\text{absorbance(control)} - \text{absorbance(rTNF-}\alpha)}$$

The cytotoxicity values (in %) are reported in graphic as a function of growing medium dilutions (using a semilogarithmic scale).

The title expressed as Unit/ml corresponds to the inverse of the dilution bringing to 50 % of cellular survival.

RESULTS

1. Neurologic-behaviouristic evaluations

In Table 4 the average score calculated at the end of the observation period in both the experiments is reported.

It is to be noted that:

- in all the control animals the presence of EAE is evident
- in the animals treated with BC-PS (on the basis of the 1st therapeutic regimen, namely from 6 hrs to 60 days) the neurologic pattern is almost normal. In particular in the 1st experiment the average score is 0.17 (vs 3.25 of the control animals); consequently BC-PS blocks the clinical symptomatology of the disease.
- in the animals treated with BC-PS wherein said treatment is suspended at the 14th day 2nd therapeutic regimen) the EAE neurologic pattern reappears: the average score (3.05) is in fact similar to that of the control animals. Therefore BC-PS effect is reversible: said datum excludes the possibility that BC-PS as such has lymphocytotoxic action.
- in the animals treated with BC-PS under clear AAE conditions, that is starting from the 16th day from AAE induction, (3rd therapeutic regimen), the neurologic pattern did not advance in its usual deterioration (the score 2.2 comprises also the situation antecedent to the treatment). Therefore BC-PS is able to reduce the seriousness and the progression of the disease, by inhibiting the disabling negative evolution.

Table 4

| Neurologic behaviouristic evaluation in mice EAE pattern: effect of the treatment with BC-PS. | | | | |
|---|---|---|---|---|
| N° of experiment | Control | Treated | Suspended | Treated at clear AAE |
| | | $1^{st}$ regimen | $2^{nd}$ regimen | 3rd regimen |
| 1 (n=15)<br>2 (n=11) | 3.25±0.56 (n=6)<br>3.8 ± 0.4 (n=5) | 0.17±0.47 (n=9)<br>0.67±0.9 (n=6) | 3.05±0.7 (n=4) | 2.2 (n=4) |

The data indicated correspond to the average score ± standard deviation (MS ± SD); the value indicated with n corresponds to the number of animals.

2. Histopathologic evaluations

In table 5 the average score calculated on the basis of the pathologic exam carried out at the level of the central nervous system (CNS) is reported.

Particularly, from the analysis conducted at the lumbo-sacral level, that is at the level of the first nervous system area wherein it is known the inflammatory phenomenon, typical of this disease, begins, it is evident that:

- in control animals, the inflammation seriousness (IS) and the axonal demyelination (DS) is high (for example the scores are respectively for animal n° 1: 3.7 and 2.0).
- in animals treated with BC-PS ($1^{st}$ regimen) neither demyelination nor inflammation is observed (for example the scores are respectively for animal n° 2: 0.1 and 0 and therefore a normal histopathologic pattern is observed.
- when the treatment with BC-PS is suspended ($2^{nd}$ regimen animal n° 5) a lesser seriousness of the inflammatory and the demyelination process is found.

Therefore the histopathologic data agree with the above reported neurologic-behaviouristic observations; a further confirmation can be deduced also from Fig. 2 and 3, relating respectively to two images of spinal cord (lumbar level) observed at the optical microscope of 1 control animal and a treated one ($1^{st}$ regimen) wherein it is noted:

- Fig. 2 (control animal): considerable presence of inflammatory cells (cellular perivascular infiltrate) and some demyelinated axons.
- Fig. 3 (animal treated with BC-PS) absence of any cellular infiltrate and of demyelination (normal pattern).

3. Biochemical evaluations: TNF levels

In figure 4 cytotoxicity data are reported (on fibroblast cultures L-929) obtained with serial dilutions of incubation media coming from lymph nodes cells. LNC (a) and splenic cells, SC (b), of the control animals and the treated ones with BC-PS (in AAE pattern).

Said percentage inhibition data of cytotoxicity indicate TNF levels produced respectively by LNC and SC in animals ± treated with BC-PS.

It is to be noted that:

- the control animals show high cellular cytotoxicity, indicating a considerable TNF concentration secreted by LNC and SC.
- the animals treated with BC-PS present a inhibited cellular cytotoxicity, indicating an inhibitory effect provoked by BC-PS against TNF production.

The percentage inhibition is about 95 % and 77 %, respectively on LNC (Fig. 4a) and SC (Fig. 4b).

The data agree with what reported about BC-PS treatment both on small mice and rabbits (item 1).

CONCLUSIONS

The whole of the data described in the 2 examples above, demonstrates that:

1. The pretreatment with phosphatidylserine (30 mg/kg/die i.p. for 3 days in mouse, or 30 mg/kg/die e.v. for 5 days in rabbit) reduces markedly (80-90 %) TNF-$\alpha$ seric levels induced by lipopolysaccharide (LPS) induction.

Said effect is dose-dependent, it requires a minimum treatment of 2 days (before LPS induction) and

persists at least 2 days from the last phosphatidylserine injection;
2. The treatment with phosphatidylserine (30 mg/kg/die i.p. x 60 days in the mouse) blocks the experimental autoimmune encephalitis on-set (A.A.E. evaluated measured with neurologic behaviouristic and hystopathologic observations) administered before the neurologic alterations: the phosphatidylserine effect does not persist; in fact, when the treatment is suspended, A.A.E. development is observed (said datum excludes the possibility that phosphatidylserine has lymphocytotoxic action).

Furthermore the treatment with phosphatidylserine is able to reduce the AAE seriousness and progression, by inhibiting the disabling negative evolution.

In the same way in animals treated with phosphatidylserine, the TNF produced by lymph node and splenic cells is markedly reduced (about 77-95 %).

TABLE 5 Hystopathologic evaluation in the AAE pattern: effect of BC-PS treatment

| Animal no. | Clinical score | day[(a)] | Treatment | Brain IS | Brain DS | Sup. spinal cord IS | Sup. spinal cord DS | Inf. spinal cord[c] IS | Inf. spinal cord[c] DS |
|---|---|---|---|---|---|---|---|---|---|
| 1 | 2.5 | 7 | Tris | 0.7±0.95 | 0 (n=3) | 1.7±0.47 | 0 (n=3) | 3.7±0.62 (n=9) | 2.0±1.05 (n=9) |
| 2 | 0 | 7 | BC-PS (1st regimen) | 0 | 0 | 0 | 0 | 0,1±0,31 (n=9) | 0±0 |
| 3 | 4 | 11 | Tris | 2,5±0.5 | 0 (n=2) | 3,3±0.47 (n=3) | 1.5±0,71 (n=3) | 3.6±0.41 (n=4) | 2.5±1.1 (n=4) |
| 4 | 0 | 11 | BC-PS (1st regimen) | 0 | 0 | 0 | 0 | 0,83+0.69 (n=6) | 0 |
| 5[b] | 2.0 | 20 | BC-PS (2nd regimen) | 1±1.4 | 0.3±0.47 | 0.67±0.47 | 0 | 2,33±0.75 | 1±1 |

IS = inflammatory scale
DS = demyelination scale
a = day when the sacrifice is conducted after AAE induction
b = treatment with BC-PS interrupted after 14 days, sample taken after 20 days within the first 24 hours of disease
c = lumbosacral level

In the compounds of formula (I) the acyl groups -COR may be the same in the different substituents or be different from each other. Acyl groups of the aliphatic series have preferably a maximum of 24 carbon atoms and may be saturated or unsaturated. A preferred class consists of the acyl groups derived from higher fatty acids, like those present in different proportion in natural phospholipids, for examples the groups derived from myristic, stearic, palmitic, lauric, oleic, linolenic, linoleic, and arachidonic acid.

A second class of acyl groups is that having a maximum of 12 carbon atoms, for example acyl coming

from: formic, acetic, propionic, valerianic, isovalerianic, trimethyl acetic acid, a butirric acid such as n-butirric acid, enantic, capronic, caprinic, pelargonic, lauric, undecylic, di-or-terbutyl acetic acid, 2 propyl valerianic acid, or from unsaturated acids such as undecylenic, angelic, tiglic acid.

The acyl groups may also derive from acids substituted by functions, such as hydroxy, aminic and carboxy groups: among them we can mention: glycolic, α-or β-hydroxy-butirric, lactic acid, glycine, alanine, valine, phenylglicine, malonic, succinic, maleic, malic acid.

The acyl of the aromatic or arylalyphatic series are those for example having only one unsubstituted benzenic ring or a substituted one with from 1 to 3 methyl or ethyl radicals, with from 1 to 3 hydroxy, methoxy or ethoxy groups, or with from 1 to 3 aminic groups or with from 1 to 3 halogen atoms.

We can mention as specific examples of said class: benzoic, salicylic, p-amino benzoic, trimethyl benzoic, tri-bromo or tri-chlorobenzoic acid.

The acyl groups may also derive from a mixed aliphatic = aromatic series such as those having only one benzene ring, optionally substituted as above reported; as specific examples phenyl acetic or phenyl propionic acid are to be cited.

Alicyclic acid from which acyl groups -COR may derive, are preferably those having 5 or 6 carbon atoms, as for example hexancarbonic or dicarbonic acid.

Among the acids of the heterocyclic series are to be mainly mentioned those having only one heterocyclic group, as the derivative of thiophene, pyrrole, thiazole, imidazole, pyridine or those of the corresponding saturated groups.

As specific acids we can cite 2- or 3- furoic, 2-thiophen acetic, nicotinic, isonicotinic. picolinic, 7-teophyllin-acetic acid.

Preferred groups of compounds of formula (I) are those wherein A is a hydrogen atom.

When B represents the group

$$\begin{array}{l} | \\ CH\text{-}O\text{-}COR \\ | \\ CH_2\text{-}OCOR \end{array}$$

the compounds have a structure corresponding to that of phosphatidylserine: in phosphatidylserine, in fact, the -COR groups derive from higher fatty acids like the above cited stearic, palmitic, oleic, linoleic, and arachidonic acids.

Also lower homologues of said phosphatidylserines may be used according to the present invention, wherein R derives from a lower aliphatic acid, namely having a maximum of 12 carbon atoms as above said. Said compounds may be prepared by following the synthetic method described in: Ingvar Lindh et al.: A general method for the synthesis of glycerophospholipids and their analogues via H-phosphonate intermediates. J. Org. Chem. 54:1338-1342 (1989).

Other preferred substance to be used according to the present invention are phosphatidylserine analogues with the preferred group above mentioned, wherein -COR groups come from the above said aromatic arylaliphatic, alicyclic or heterocyclic series, and also said compounds may be prepared by known methodologies, for example by using the same synthetic method utilized for preparing derivatives containing aliphatic acids as above cited.

Other phosphatidylserine analogues which can be prepared with a synthetic method and comprised in the general formula (I) are described in EPA-0396080 published on 07.11.90, and other ones comprised in the same formula which may be prepared analogously.

Among the salts of the compound of formula (I) the metal salts are particularly important, and especially, the alkali metal salts, like those of sodium and potassium.

However, alkali earth salts may also be used according to the present invention like calcium or magnesium salts.

The compounds of formula (I) may also be used in the form of acid addition salts, for example in the form of hydro-chlorides hydrobromides, sulfonates sulfates or pharmaceutically acceptable salts of strong organic acids.

The present invention relates also to pharmaceutical preparations containing the active compounds of formula (I) to be used in said treatments.

These preparations may be used in man or in animals.

They may be administered by oral or parenteral route, preferably by intramuscular, intravenous injection or by infusion.

They may be in the form of solutions (or lyophylized powders) associated with one or more pharmaceutically acceptable carriers or diluents and contained in media which are buffered at a suitable pH and isotonic with physiologic fluids.

The pharmaceutical compositions according to the present invention may preferably have a content of the active principle comprised between 25 and 500 mg associated with one or more pharmaceutically acceptable excipients.

The dose of the active principle administered will depend on the desired effects and on the chosen administration route and it may be comprised between about 0.5 and 12 mg/kg/pro dose, equivalent to a dose comprised between about 50 and 800 mg/pro dose (till a maximum of 3 times a days).

For illustrative, but not limitative purposes we report herewith some examples of the pharmaceutical compositions according to the present invention.

The following examples illustrate the invention:

**Example 1:**

Preparation of the BC-PS composition

333 g of phosphatidylethanolamine (PE) having a title higher than 83 % were added to a solution of 1000 g of phosphatidylserine (PS) extracted from bovine nervous tissue having a title higher than 95 %, in 5 liters of a methanol-chloroform (2:1) mixture.

The solution obtained was stirred for 30 minutes, filtered through a sterilizing membrane and precipitated with 5 volumes of acetone under suitable stirring.

The precipitate was separated and dried under vacuum at low temperature.

In this way about 1250 g of the composition defined as BC-PS were obtained.

**Example 2**

A 2 ml vial contains:

| | |
|---|---|
| - phosphatidylserine | 50.00 mg |

Other components:

| | |
|---|---|
| - lecithin | 15.00 mg |
| - mannitol | 100.00 mg |
| - bibasic sodium phosphate -$12H_2O$ | 2.26 mg |
| - monobasic sodium phosphate -$2H_2O$ | 2.14 mg |
| - water p.f.i. q.s. to | 2 ml |

**Example 3**

A 2 ml vial contains:

| | |
|---|---|
| - phosphatidylserine | 50.00 mg |

Other components:

| | |
|---|---|
| - mannitol | 100.00 mg |
| - bibasic sodium phosphate -$12H_2O$ | 2.26 mg |
| - monobasic sodium phosphate -$2H_2O$ | 2.14 mg |
| - water p.f.i. q.s. to | 2 ml |

**Example 4**

A 10 ml bottle wherein every ml contains:

| | |
|---|---|
| - phosphatidylserine | 25.00 mg |

Other components:

| | |
|---|---|
| - lecithin<br>- mannitol<br>- bibasic sodium phosphate -12$H_2O$<br>- monobasic sodium phosphate -2$H_2O$<br>- water p.f.i. q.s. to | 7.50 mg<br>50.00 mg<br>1.13 mg<br>1.07 mg<br>1 ml |

**Example 5**

A 10 ml bottle wherein every ml contains:

| | |
|---|---|
| - phosphatidylserine | 25.00 mg |

Other components:

| | |
|---|---|
| - mannitol<br>- bibasic sodium phosphate -12$H_2O$<br>- monobasic sodium phosphate -2$H_2O$<br>- water p.f.i q.s. to | 50.00 mg<br>1.13 mg<br>1.07 mg<br>1 ml |

**Example 6**

A capsule contains:

| | |
|---|---|
| - phosphatidylserine | 100.00 mg |

Other components:

| | |
|---|---|
| - soy lecithin<br>- vegetable oil | 30.00 mg<br>270.00 mg |

The external coating is composed by:

| | |
|---|---|
| - gelatine<br>- glycerol<br>- brown ferric oxide E 172<br>- red ferric oxide E 172<br>- sodium ethyl-para-hydroxybenzoate<br>- sodium propyl-para-hydrobenzoate | 129.00 mg<br>49.00 mg<br>1.10 mg<br>0.40 mg<br>0.20 mg<br>0.10 mg |

**Example 7**

A capsule contains:

| | |
|---|---|
| - phosphatidylserine | 100.00 mg |

Other components:

| | |
|---|---|
| - vegetable oil | 270.00 mg |

The external coating is composed by:

| | |
|---|---|
| - gelatine | 129.00 mg |
| - glycerol | 49.00 mg |
| - brown ferric oxide E 172 | 1.10 mg |
| - red ferric oxide E 172 | 0.40 mg |
| - sodium ethyl-para-hydroxybenzoate | 0.20 mg |
| - sodium propyl-para-hydrobenzoate | 0.10 mg |

**Example 8**

A small bottle together with a solvent vial for parenteral administration wherein every small bottle contains:
Lyophilized active component

| | |
|---|---|
| - phosphatidylserine | 50.00 mg |

Other components:

| | |
|---|---|
| - lecithin | 15.00 mg |
| - mannitol | 100.00 mg |
| - water p.f.i. q.s. to | 4 ml |

**Example 9**

A small bottle together with a solvent vial for parenteral administration wherein every small bottle contains:
Lyophilized active component

| | |
|---|---|
| - phosphatidylserine | 50.00 mg |

Other components:

| | |
|---|---|
| - mannitol | 100.00 mg |
| - water p.f.i. q.s. to | 4 ml |

**Example 10**

A small single-dose bag (water-dispersable granules for oral use) contains:

| | |
|---|---|
| - phosphatidylserine | 100.00 mg |

Other components:

| | |
|---|---|
| - sodium ascorbate | 5.00 mg |
| - aspartame | 5.00 mg |
| - colloidal silica | 10.00 mg |
| - soy lecithin | 30.00 mg |
| - natural flavour | 40.00 mg |
| - mannitol | 350.00 mg |
| - fructose q.s. to | 1.50 g |

**Example 11**

A small single-dose bag (water-dispersable granules for oral use) contains:

| | |
|---|---|
| - phosphatidylserine | 100.00 mg |

Other components:

| | |
|---|---|
| - sodium ascorbate | 5.00 mg |
| - aspartame | 5.00 mg |
| - colloidal silica | 10.00 mg |
| - natural flavour | 40.00 mg |
| - mannitol | 350.00 mg |
| - fructose q.s. to | 1.50 g |

**Example 12**

A small single-dose bag (water-dispersable granules for oral use) contains:

| | |
|---|---|
| - phosphatidylserine | 200.00 mg |

Other components:

| | |
|---|---|
| - sodium ascorbate | 10.00 mg |
| - aspartame | 10.00 mg |
| - colloidal silica | 20.00 mg |
| - soy lecithin | 60.00 mg |
| - natural flavour | 80.00 mg |
| - mannitol | 700.00 mg |
| - fructose q.s. to | 3.00 g |

**Example 13**

A small single-dose bag (water-dispersable granules for oral use) contains:

| | |
|---|---|
| - phosphatidylserine | 200.00 mg |

Other components:

| | |
|---|---|
| - sodium ascorbate<br>- aspartame<br>- colloidal silica<br>- natural flavour<br>- mannitol<br>- fructose q.s. to | 10.00 mg<br>10.00 mg<br>20.00 mg<br>80.00 mg<br>700.00 mg<br>3.00 g |

**Example 14**

A 2 ml vial contains:

| | |
|---|---|
| $PSS_4$<br>- monobasic sodium phosphate<br>- bibasic sodium phosphate<br>- apyrogen bidistilled water q.s. to | 40.0 mg<br>2.14 mg<br>2.26 mg<br>2 ml |

**Example 15**

A 3 ml vial contains:

| | |
|---|---|
| - $PSS_4$<br>- monobasic sodium phosphate<br>- bibasic sodium phosphate<br>- mannitol<br>- apirogenic bidistilled water q.s. to | 80.0 mg<br>3.21 mg<br>3.39 mg<br>30 mg<br>3 ml |

**Example 16**

A gelatine capsule contains:

| | |
|---|---|
| - $PSS_4$<br>- vegetable oil<br>- beeswax | 120 mg<br>270 mg<br>1 mg |

**Example 17**

A gelatine capsule contains:

| | |
|---|---|
| - $PSS_4$<br>- vegetable oil<br>- beeswax | 320 mg<br>270 mg<br>1 mg |

**Example 18**

A coated tablet contains:

| | |
|---|---|
| - $PSS_4$ | 60 mg |
| - mannitol | 100 mg |
| - microcrystalline cellulose | 25 mg |
| - amid | 5 mg |
| - saccharose | 30 mg |
| - film coat | 5 mg |

**Example 19**

An operculum contains:

| | |
|---|---|
| - $PSS_4$ | 185 mg |
| - mannitol | 100 mg |
| - lactose | 100 mg |

**Claims**

**1.** The use of a compound of formula (I)

(I) 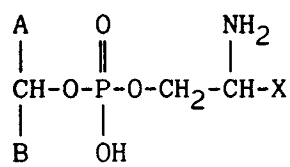

wherein X = H, -COOH;
A = H, $-(CH_2)_n$-O-CO-R with n = 1,2,

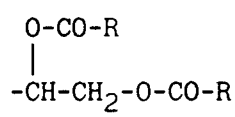

and B = $(CH_2)_m$ -OY with Y = H or -CO-R
and m = 1,2,3,4,

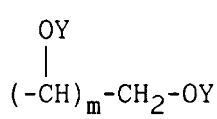

with Y = H or -CO-R
and m = 1,2,3, provided that when A is different from H, m = 1,2 and wherein the various R groups may be equal or different from each other and the corresponding -COR groups represent acylic radicals of aliphatic-, aromatic-, alicyclic- or heterocyclic- series acids, or of a mixture of compounds having the above formula but differing from each other for the different meanings of R, when the radical - COR derives from higher aliphatic acids as it generally occurs in natural phospholipids, or of their acid addition salts or metal salts in the preparation of pharmaceutical compositions active in the prophylaxis and treatment of degenerative pathologies also associated with autoimmune disfunctions, wherein the so called "tumor necrosis factor" (TNF) is involved.

2. The use as claimed in claim 1, wherein a phosphatidylserine isolated from bovine brain is administered.

3. The use, as claimed in claim 1, wherein a phosphatidylserine consisting of a mixture of compounds corresponding to the following formula is administered

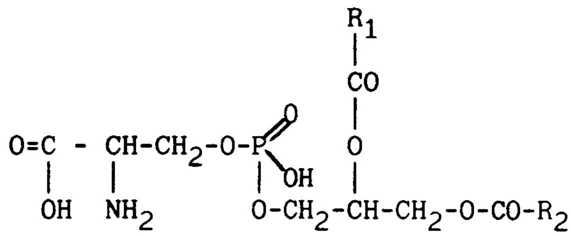

wherein -CO-$R_1$ and -CO-$R_2$ are acyl radicals deriving from myristic stearic, palmitic, lauric, oleic, linolenic, linoleic, arachidonic acid.

4. The use, as claimed in claim 1, wherein phosphatidylserine known as phosphatidylserine BC-PS is administered.

5. The use, as claimed in claim 1, wherein phosphatidylethanolamine (PE) is administered.

6. The use, as claimed in claim 1, wherein lisophosphatidylserine (DPS) is administered.

7. The use, as claimed in claim 1, wherein 1-myristoyl-2-deoxi-3-glycerophospho-L-serine ($PSS_4$) is administered.

8. The use, as claimed in claim 1, wherein alkaline salts of the corresponding phospholipids of formula (I) are administered.

9. The use, as claimed in claim 8, wherein sodium and potassium salts of the compounds of formula (I) are used.

10. The use, as claimed in claim 1, wherein a compound of formula (I) is administered, in which
A = H,

B= 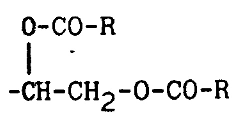

and -CO-R represent acyl radicals deriving from acids of the aliphatic series having a maximum of 12 carbon atoms and wherein the two acyl groups are equal to each other.

11. The use, as claimed in claim 1, wherein the compound of formula (I) is administered having
A = H,

B= 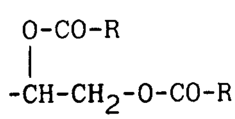

in which -CO-R represent acyl radicals deriving from aromatic or aryl aliphatic series and having only one benzenic group unsubstituted or substituted with from 1 to 3 methyl or ethyl groups, with from 1 to 3 hydroxy, methoxy or ethoxy groups or with from 1 to 3 aminic groups or with from 1 to 3 halogen atoms.

**12.** The use, as claimed in claim 1, wherein a compound of formula (I) is administered, having
A = H

$$\begin{array}{ll} & \quad O-CO-R \\ & \quad | \\ B= & -CH-CH_2-O-CO-R \end{array}$$

in which the -CO-R groups represent acyl radicals deriving from acids of the heterocyclic series with only one heterocyclic group selected from a radical of thiophene, pyrrole, thiazole, imidazole, pyridine or of their corresponding saturated groups.

**13.** The use of a compound, as claimed in one of the preceding claims for the multiple sclerosis treatment.

**14.** The use, as claimed in claim 13, wherein a phosphatidylserine is administered.

**15.** The use, as claimed in one of the preceding claims, for the treatment of cachectic states associated with infections and neoblastic diseases.

**16.** The use, as claimed in one of the preceding claims, for the pneumosclerosis treatment.

**17.** The use, as claimed in one of the preceding claims for the rheumatoid arthritis treatment.

**18.** The use, as claimed in one of the preceding claims, for the treatment of bacterial, protozoal and "human immunodeficiency virus" (HIV) infections.

**19.** The use, as claimed in one of the preceding claims, for the treatment of cerebral complications of malaria.

**20.** The use as claimed in one of the preceding claims, for the treatment of insulino-dependent diabetes.

**21.** The use, as claimed in one of claims 15-20 wherein a phosphatidylserine is administered.

**22.** The use, as claimed in one of the preceding claims, in human medicine.

**23.** The use, as claimed in one of the preceding claims, in veterinary medicine.

**24.** A pharmaceutical composition for the use mentioned in claims 1, containing a compound of formula (I) as the active ingredient combined with one or more pharmaceutically acceptable excipients, carriers or diluents.

**25.** A pharmaceutical composition, as claimed in claim 24, suitable for oral administration.

**26.** A pharmaceutical composition, as claimed in claim 24, suitable for parenteral administration.

**27.** A pharmaceutical composition, as claimed in claim 26, suitable for intramuscular, intravenous administration or for infusion.

**28.** A pharmaceutical composition as claimed in one of the claims 26 and 27, under the form of a solution of the active compound or in the form of lyophilized powder to be associated with one or more diluents contained in media which are buffered at suitable pH and isotonic with physiologic fluids.

**29.** The use as claimed in claim 1, wherein the pharmaceutical compositions have a content of active principale comprised between 25 and 500 mg.

**30.** A pharmaceutical composition as claimed in claim 24, wherein the content of active principle is comprised between 25 and 500 mg.

FIG. 1

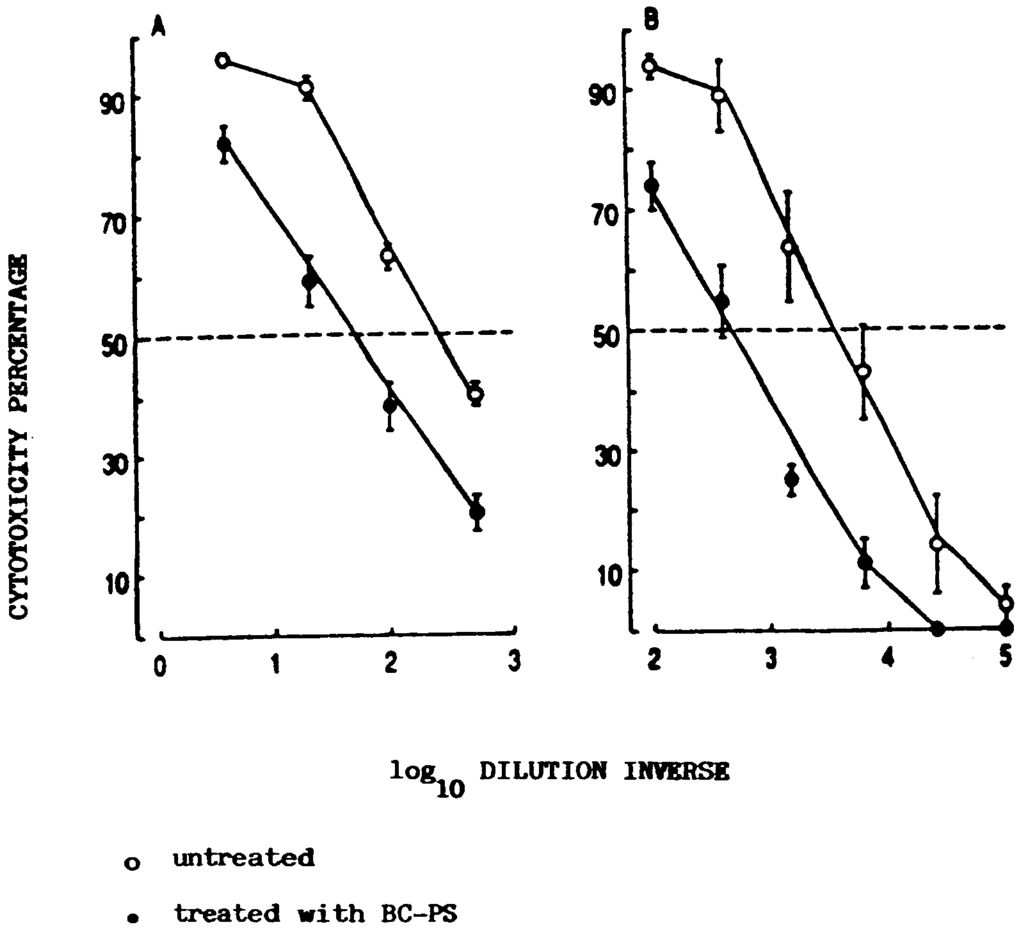
A
B
CYTOTOXICITY PERCENTAGE
90
70
50
30
10
0
1
2
3
4
5
log10 DILUTION INVERSE
o untreated
• treated with BC-PS

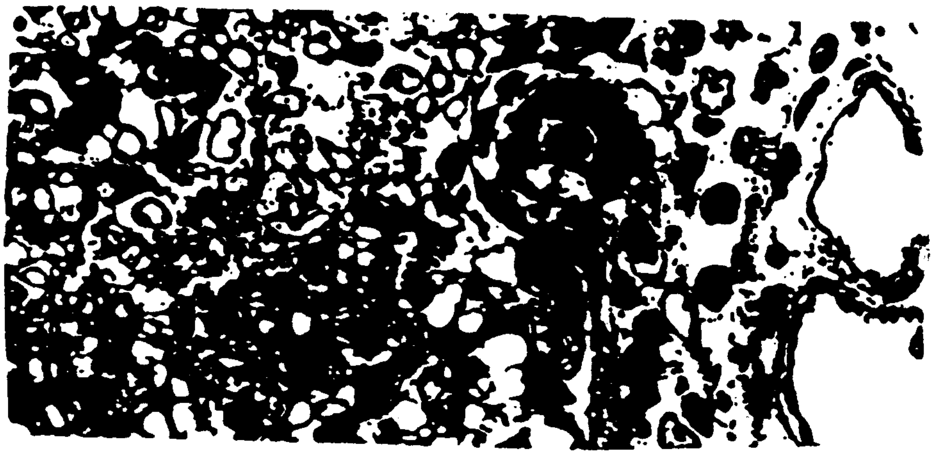

FIG. 2

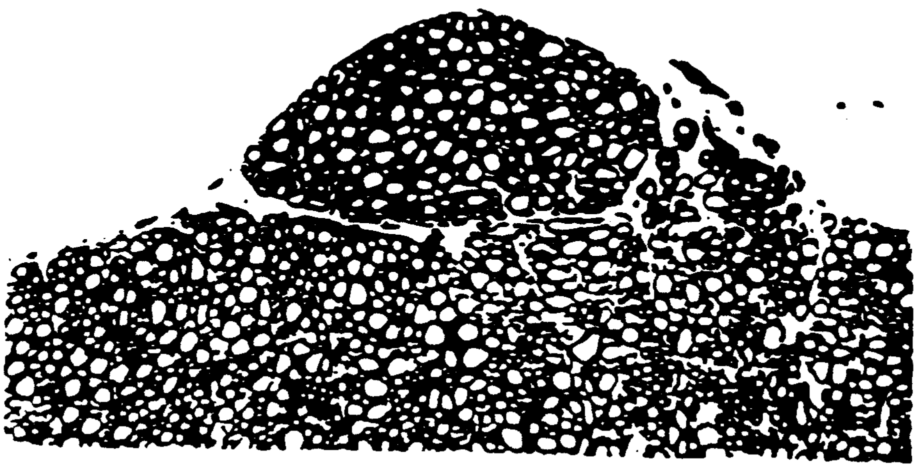

FIG.3

FIG. 4

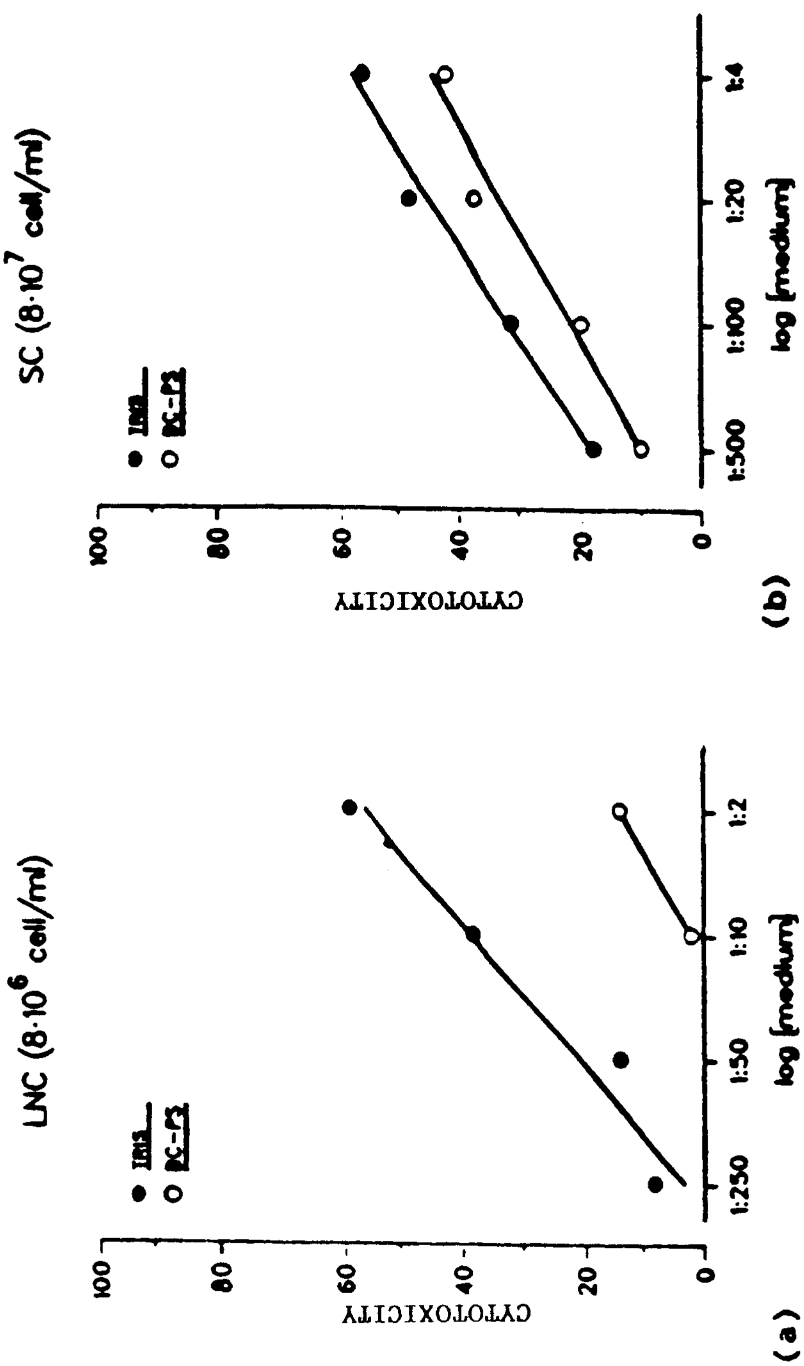
LNC (8·10^6 cell/ml)
SC (8·10^7 cell/ml)
CYTOTOXICITY
log [medium]
1:250
1:50
1:10
1:2
1:500
1:100
1:20
1:4
0
20
40
60
80
100
(a)
(b)

European Patent Office

# EUROPEAN SEARCH REPORT

Application Number

EP 92 10 4039

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 901 777 (MACNAUGHT PTY. LTD) 9 March 1989<br>* claims 1,2,7,17 *<br>--- | 1-12,17, 21-23, 24-30 | A61K31/66 |
| D,X | EP-A-0 148 045 (FIDIA SPA) 10 July 1985<br>* page 12 - page 14 *<br>----- | 24-28,30 | |
| | | | TECHNICAL FIELDS SEARCHED (Int. Cl.5) |
| | | | A61K |

The present search report has been drawn up for all claims

| Place of search | Date of completion of the search | Examiner |
|---|---|---|
| MUNICH | 02 JULY 1992 | TZSCHOPPE D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)